# EUROPÄISCHE PATENTSCHRIFT

(11) **EP 0 435 028 B1**
(45) Veröffentlichungstag und Bekanntmachung des Hinweises auf die Patenterteilung: **06.03.1996**
(21) Anmeldenummer: 90123287.6
(22) Anmeldetag: 15.12.1990
(51) Int. Cl.: C12P 7/62, C12N 15/33, C12N 1/06, C12N 15/55

(54) **Verfahren zur Freisetzung von Poly-3-Hydroxycarbonsäuren**
Procedure for releasing poly-3-hydroxycarboxylic acids
Procédé de libération des acides poly-3-hydroxycarboniques

(30) Priorität: 27.12.1989 AT 2942/89
(43) Veröffentlichungstag der Anmeldung: 03.07.1991
(73) Patentinhaber: PCD-Polymere Gesellschaft m.b.H., A-2323 Schwechat-Mannswörth (AT)
(72) Erfinder: Lubitz, Werner, Dr., W-8000 München 2 (DE)
(74) Vertreter: Kunz, Ekkehard, Dr.

(56) Entgegenhaltungen:
- EP-A- 0 291 021
- CHEMICAL ABSTRACTS, Band 112, 26. Februar 1990, Seite 261, Zusammenfassung Nr. 71599u, Columbus, Ohio, US; R.L. DABORA et al.: "Release of beta- galactosidasefrom E. coli by a plasmid containing a temperature sensitive lytic function",& BIOTECHNOL. LETT. 1989, 11(12), 845-50
- JOURNAL OF BACTERIOLOGY, Band 170, Nr. 10, Oktober 1988, Seiten 4431-4436, Baltimore, US; S.C. SLATER et al.: "Cloning and expression in Escherichia coli
- of the Alcaligenes eutrophus H16 poly-beta-hydroxybutyrate biosyntheticpathway"
- CHEMICAL ABSTRACTS, Band 198, 1983, 17. Januar 1983, Seite 148, ZusammenfassungNr. 12359k, Columbus, Ohio, US; K.D. YOUNG et al.: "Lytic action of clonedphiX174 gene E", & J. VIROL. 1982, 44(3), 993-1002
- J. Virol. 1982, 44(3), 993 - 1002
- Biotechnol. Lett. 1989, 11(12), 845 - 50

## Beschreibung

Die Erfindung betrifft ein Verfahren zur Freisetzung von Homo- und/oder Copolymeren von 3-Hydroxycarbonsäuren aus diese enthaltenden Zellen gram-negativer Bakterien durch induzierte Lyse mit Hilfe eines natürlichen oder chimären Lysegens.

Unter Homo- und/oder Copolymeren von 3-Hydroxycarbonsäuren sind solche zu verstehen, die von gram-negativen Bakterien, wie beispielsweise Alcaligenes, Athiorhodium, Pseudomonas, Rhizobium, Spirillum gebildet und gespeichert werden können. Insbesondere sind dies Homo- und/oder Copolymere der 3-Hydroxybuttersäure (PHB) und der 3-Hydroxyvaleriansäure (PHV). Es ist aber auch möglich, daß diese Bakterien andere Poly-3-Hydroxycarbonsäuren speichern. Verfahren zur Bildung und Speicherung von PHB und PHV sind beispielsweise in EP-A-0 015 669, EP-A-0 046 344 und EP-A-0 052 459 beschrieben.

Aus Slater et al., J Bacteriol. 170,4431 ff (1988) ist bekannt, daß die für die PHB-Erzeugung in der Zelle verantwortlichen Enzyme in Bakterienzellen, die nicht PHB erzeugen, beispielsweise in Escherichia coli kloniert und mit Hilfe entsprechender Plasmide exprimiert werden können, wobei die klonierten Enzyme dann die Synthese von PHB in der Zelle hervorrufen.

Die Freisetzung von Poly-3-Hydroxycarbonsäuren aus den Zellen der Mikroorganismen kann durch Extrahieren des Polymeren aus den trockenen Zellen mit einem Extraktionsmittel, in dem dieses gut löslich ist, erfolgen. Ein solches Verfahren ist beispielsweise in EP-A 15 123 beschrieben. Dabei müssen die Zellen vorerst aufgebrochen und so für das entsprechende Extraktionsmittel durchlässig gemacht werden. Ein Nachteil dieser Verfahren liegt darin, daß durch das Extraktionsmittel nicht nur das Polymer aus der Zelle gelöst wird, sondern auch bestimmte Zellbestandteile, im speziellen Lipide, in Lösung gehen und diese Verunreinigungen anschließend vom Polymer getrennt werden müssen.

Eine Möglichkeit der Freisetzung von Poly-3-Hydroxycarbonsäuren aus feuchten Zellen ist in der EP-A 145 223 beschrieben. Dabei wird das Zellmaterial enzymatisch und/oder durch oberflächenaktive Substanzen in Lösung gebracht, wobei das Polymer ungelöst zurückbleibt. Die Auflösung des Zellmaterials muß dabei in mehreren Stufen erfolgen, wobei auch dann noch die Auflösung unvollständig bleibt und nicht gelöste Zellbestandteile im Polymer zurückbleiben.

Es ist auch bekannt, gram-negative Bakterien durch Expression klonierter Lysegene aufzuschließen. In DE-A 37 15 840 ist beispielsweise die Lyse von E. coli-Zellen mit Hilfe eines chimären Lysegens, das durch einen temperaturinduzierten Promotor gesteuert wird, beschrieben, wobei die Zellen innerhalb von etwa 30 bis 70 Minuten lysiert werden.

In J. Virol, 1982, 44(3), 993-1002 (Chemical Abstract Bd. 198, 1983, Nr. 12359k) ist beschrieben, daß in gram negativen Zellen, wie E. coli Zellen, durch Expression des klonierten Lysegens E des Bakteriophagen Phi X174 Sphäroblasten gebildet werden können, wobei vor der Induktion der Expression des Lysegens durch Zufügen von MgSO₄ eine Ionenkonzentration von 0,2 mol/l eingestellt wird. In Biotechnol. Letter 1989, 11(12), 845 bis 850 (Chemical Abstracts Bd. 112,1990, Nr. 71599u) ist die Induktion der Expression des Lysegens E aus dem Bakteriophagen Phi X174 durch Temperaturerhöhung zum Zweck der Freisetzung von beta-Galaktosidase aus E. coli Zellen offenbart. Zur Erhöhung der Ausbeute ist eine Ultraschallbehandlung im Anschluß an die Lyse beschrieben. Aus Witte und Lubitz, Eur. J. Biochem. 180, 393ff (1989) geht allerdings hervor, daß sich bei der Lyse von E. coli Zellen mit Hilfe des Lysegens E des Bakteriophagen Phi X174 nur ein kleiner transmembraner Lysetunnel mit einem Durchmesser von etwa 30 bis 100 nm bildet, durch den Zellinhaltsstoffe in das umgebende Medium diffundieren können. Die Zellen werden innerhalb von etwa 30 bis 60 Minuten lysiert. Da dieser Tunnel sehr klein ist, ist die Anwendung dieses Verfahrens zur Freisetzung von Polymeren, deren Größe beinahe das gesamte Zellvolumen ausfüllt, in größeren Maßstab zu langwierig.

Es konnte nun ein Verfahren zur Freisetzung von Poly-3-Hydroxycarbonsäurugranula aus diese enthaltenden Zellen gram-negativer Bakterien durch Expression eines klonierten natürlichen oder chimären Lysegens gefunden werden, bei dem überraschenderweise nicht kleine Lysetunnel, sondern Öffnungen gebildet werden, die in ihrem Durchmesser beinahe den gesamten Querschnitt der Zelle umfassen, bei dem die Zellhüllen in ihrer ursprünglichen Gestalt bestehen bleiben, und bei dem die in der Zellwand verankerten Lipide in der Zelle bleiben, wobei eine vollständige Abtrennung der Poly-3-Hydroxycarbonsäuregranula in wäßrigem Medium möglich ist.

Gegenstand der Erfindung ist demnach ein Verfahren zur Freisetzung von Poly-3-Hydroxycarbonsäuregranula aus diese enthaltenden Zellen gram-negativer Bakterien durch Expression eines klonierten natürlichen oder chimären Lysegens, das dadurch gekennzeichnet ist, daß man vor der Induktion der Lysegen Expression die Ionenkonzentration an zweiwertigen Kationen auf 0,05 bis 0,5 mol/l erhöht, die Expression des Lysegens durch Temperaturerhöhung induziert, die Zellen erntet und die noch feuchten Zellen in Wasser oder Pufferlösungen resuspendiert, wodurch die Freisetzung der Polymergranula durch spontane Lyse erfolgt.

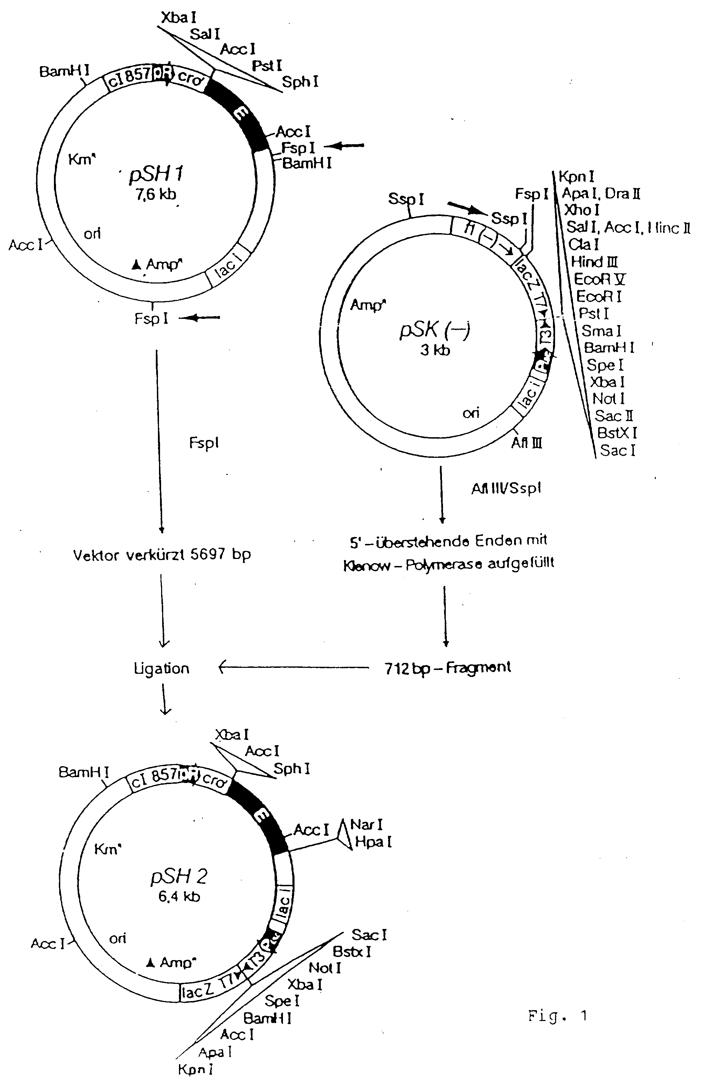

Zur Durchführung des Verfahrens werden die gram-negativen Bakterien, welche die Poly-3-Hydroxycarbonsäuren erzeugen, durch Expression eines geeigneten klonierten Lysegens zur Lyse programmiert. Das Lysegen kann ein natürliches, beispielsweise das Lysegen E der Bakteriophagen PhiX174, oder ein chimäres Lysegen sein. Vorzugsweise wird das klonierte Lysegen E der Bakteriophagen PhiX174, beispielsweise auf Plasmid pSH2 (Fig 1) oder pAW13 verwendet. Zur Herstellung von Plasmid pAW13 wird das PsH/BamHI Fragment aus Plasmid pSB12 (Blasi et al, J. Gen. Microbiol. 131, (1985), 1107ff), das das Gen E unter Kontrolle des Lambda pL-Promotors enthält, in die PsH/BamHI Schnittstellen des pACYC177 (Chang et al. J. Bacteriol 134 (1978), 1141ff) eingefügt. Anschließend wird das erhaltene Plasmid mit PstI geschnitten, worauf es mit T4DNA Polymerase behandelt wird, um das bla-Gen zu zerstören. Das so erhaltene Plasmid pAW13 trägt das Lysegen. E unter Kontrolle des Lambda pL Promotors. Poly-3-Hydroxycarbonsäuren erzeugende gram-negative Bakterien, die zur Lyse programmiert werden können, sind beispielsweisenes, Athiorhodium, Pseudomonas, Rhizobium, Spirillium sowie Escherichia coli, in die PHB/PHV produzierende Enzyme kloniert sind.

Die Bakterienzellen werden auf übliche Weise fermentiert, d.h. auf einem für die Bildung von Poly-3-Hydroxycarbonsäuren geeigneten Substrat, beispielsweise mit Kohlehydraten als Energiequelle, in Abhängigkeit vom eingesetzten Bakterium bei einer Temperatur von etwa 28 bis 37 °C kultiviert. Dabei ist zu beachten, daß die Temperatur so niedrig gehalten wird, daß der temperatursensitive Promotor des Lysegens die Lyse nicht schon während der Fermentation induziert.
Anschließend wird die Konzentration zweiwertiger Kationen, beispielsweise durch Zugabe von MgSO₄, CaCO₃ u. dgl. auf etwa 0,05 bis 0,5 mol/l, vorzugsweise auf etwa 0,1 bis 0,3 mol/l erhöht. Nun wird die Lyse durch Erhöhung der Temperatur auf etwa 42 °C induziert, ihr Einsetzen wird durch die hohe Ionenkonzentration zweiwertiger Kationen noch verhindert. Die Zellen werden durch sanftes Zentrifugieren geerntet und das feuchte Zellmaterial anschließend in Wasser oder wäßrigen Pufferlösungen resuspendiert, wobei es zu einem spontanen Aufplatzen der Zellen kommt und cytoplasmatische Inhaltsstoffe und darin enthaltene Poly-3-Hydroxycarbonsäuregranula innerhalb weniger Minuten freigesetzt werden. Als wäßrige Pufferlösungen können beispielsweise 0,001 - 0,01 mol/l TrisHCl, 0,001 - 0,01 mol/l KH₂/K₂HPO₄ - Puffer und dgl., eingesetzt werden.
Die freigesetzten Polymergranula, die cytoplasmatischen Inhaltsstoffe und die leeren Zellhüllen können durch übliche Trennmethoden, beispielsweise durch Dichtegradientenzentrifugation getrennt werden.

### Beispiel 1:

### Konstruktion des Klonierungsvektors pSH2 (Fig. 1)

Das Plasmid pSH1 (S. Haist, PhiX174 Spezifische Vektorkonstruktionen, Diplomarbeit, Ludwig-Maximilian-Universität München, August 1989) wurde mit dem Enzym Fsp I im entsprechenden Restriktionspuffer geschnitten, wobei ein 5697 bp und ein 1969 bp großes Fragment mit glatten Enden entstand. Die beiden Fragmente wurden gelelektrophoretisch getrennt und mittels "Gene Clean" (Fa. Bio 101 Inc.) gereinigt.

Das Plasmid pBluescript pSK(-) (Fa. Stratagene) wurde mit den Restriktionsenzysmen SspI, das glatte Enden bildet, und Afl III, das 5' überstehende Enden bildet, im entsprechenden Restriktionspuffer geschnitten, wobei drei Fragmente mit 1697 bp, 712 bp und 555 bp entstanden. Das 712 bp große Fragment wurde mit "Gene Clean" eluiert und das 5'überstehende Ende mit Klenow Polymerase aufgefüllt. Das 5697 bp große Fragment aus pSH1 wurde mit diesem 712 bp großen Fragment mit Hilfe von T4DNA Ligase im entsprechenden Puffer ligiert.

### Beispiel 2:

### Transformation von E.coli PC 1363 (Phabagen Collection, Universität Utrecht)

Die Transformation des E.coli PC 1363 erfolgt in an sich bekannter Weise, beispielsweise nach der in Maniatis et al. Molecular Cleaning, Gold Spring Harbor Laboratory, New York (1982) beschriebenen Methode.

Dazu werden 5 ml Lurea Broth, ein Nährmedium bestehend aus 10 g Pepton, 5 g Hefeextrakt, 5 g NaCl in 1000 ml destilliertem Wasser (pH 7) mit 1 ml Vorkultur von E.coli PC 1363 inokuliert und bei 37°C geschüttelt. Die Zellen werden durch Zentrifugation geerntet und in 25 ml Transfomationspuffer bestehend aus 150 mmol/l KCl, 50 mmol/l MgCl₂ und 1 mmol/l TrisHCl in Eis suspendiert. Nach erneuter Zentrifugation werden die Zellen in 2 ml Transformationspuffer resuspendiert. Von dieser Suspension werden 0,1 ml mit Plasmid DNA (pSH2 (p15A) und pSB20-PHB (pHB1) (Slater et al, J.Bacteriol. 170, p 4431ff (1988))) versetzt und nach etwa 1 Stunde 1,2 ml Lurea Broth bei 28°C zugesetzt. Die Transformanten wurden entsprechend der Antibiotikaresistenzmarker der Plasmide selektioniert.

### Beispiel 3:

### Fermentation und Lyse von E.coli PC 1363 (pSB20, pSH2)

10 ml Lurea Broth wurden mit einer Übernachtkultur des nach Beispiel 2 hergestellten E.coli Stammes beimpft und bei 28 °C bis zu einer optischen Dichte OD₆₀₀ = 0.2 - 0.9 kultiviert. Dann wurde eine 2 mol/l MgSO₄-Lösung bis zu einer Endkonzentration von 0,2 mol zugesetzt und 30 min bei 28 °C inkubiert. Die Temperatur wurde anschließend auf 42 °C erhöht, nach 30 min wurden die Zellen durch Zentrifugation (7000 U/min, 5 min) bei 4 °C geerntet und das Zellmaterial in 10 ml destilliertem Wasser resuspendiert. Die Lyse erfolgt während der Suspension innerhalb von 10 min.

Zum lysierten Zellgemisch wurde Saccharose bis zu einer Endkonzentration von 55 % zugesetzt und mit 50, 45 und 40 %iger Saccharoselösung in 3 mmol/l EDTA überschichtet. Nach 16 Stunden Zentrifugation (4 °C, 37 000 U/min, SW41Ti) wurde der Gradient fraktioniert und der Proteingehalt, die Dichte sowie der Gehalt an PHB/PHV bestimmt. Es wurden drei Fraktionen mit Dichten 1,24 g/ml (nichtlysierte Zellen), 1,22 g/ml (Zellghosts) und 1,20 g/ml (PHB/PHV) erhalten. Dabei wurden 60 bis 80 % PHB/PHV-Granula freigesetzt.

## Patentansprüche

1. Verfahren zur Freisetzung von Poly-3-Hydroxycarbonsäuregranula aus diese enthaltenden Zellen gram-negativer Bakterien durch Expression eines klonierten natürlichen oder chimären Lyse-Gens, dadurch gekennzeichnet, daß man vor der Induktion der Lysegen Expression die Ionenkonzentration an zweiwertigen Kationen auf 0,05 bis 0,5 mol/l erhöht, die Expression des Lysegens durch Temperaturerhöhung induziert, die Zellen erntet und die noch feuchten Zellen in Wasser oder Pufferlösungen resuspendiert, wodurch die Freisetzung der Polymergranula durch spontane Lyse erfolgt.

2. Verfahren nach Anspruch 1, dadurch gekennzeichnet, daß als Lysegen das klonierte Lysegen E der Bakteriophagen Phi X174 eingesetzt wird.

3. Verfahren nach einem der Ansprüche 1 oder 2, dadurch gekennzeichnet, daß die Konzentration an zweiwertigen Kationen auf 0,1 bis 0,3 mol/l erhöht wird.

4. Verfahren nach einem der Ansprüche 1 bis 3, dadurch gekennzeichnet, daß die Konzentration an zweiwertigen Kationen durch Zufügen von MgSO₄ oder CaCO₃ erhöht wird.

5. Verfahren nach einem der Ansprüche 1 bis 4, dadurch gekennzeichnet, daß als gram-negative Bakterien Alcaligenes oder E. coli, in die Enzyme, die Homo- und/oder Copolymere der 3-Hydroxybuttersäure und/oder der 3-Hydroxyvaleriansäure produzieren, kloniert sind, eingesetzt werden.

6. Verfahren nach Anspruch 5, dadurch gekennzeichnet, daß als gram-negative Bakterien E. coli, in die Enzyme, die Homo-und/oder Copolymere der 3-Hydroxybuttersäure und/oder der 3-Hydroxyvaleriansäure produzieren, kloniert sind, eingesetzt werden.

## Claims

1. Process for releasing poly(3-hydroxy carboxylic acid) granules from cells of Gram-negative bacteria containing the latter, by expression of a cloned natural or chimeric lysis gene, characterised in that, before induction of lysis gene expression, the ionic concentration of doubly charged cations is increased to from 0.05 to 0.5 mol/l, the expression of the lysis gene is induced by raising the temperature, the cells are harvested, and the still wet cells are resuspended in water or buffer solutions, which results in the release of the polymer granules by spontaneous lysis.

2. Process according to Claim 1, characterised in that the lysis gene employed is the cloned lysis gene E of bacteriophages Phi X174.

3. Process according to either of Claims 1 or 2, characterised in that the concentration of doubly charged cations is raised to from 0.1 to 0.3 mol/l.

4. Process according to any of Claims 1 to 3, characterised in that the concentration of doubly charged cations is raised by adding MgSO₄, or CaCO₃.

5. Process according to any of Claims 1 to 4, characterised in that Alcaligenes or E. coli into which enzymes which produce homo- and/or copolymers of 3-hydroxybutyric acid and/or 3-hydroxyvaleric acid are cloned are employed as Gram-negative bacteria.

6. Process according to Claim 5, characterised in that E. coli into which enzymes which produce homo-and/or copolymers of 3-hydroxybutyric acid and/or 3-hydroxyvaleric acid are cloned are employed as Gram-negative bacteria.

## Revendications

1. Procédé de libération de granules d'acides poly-3-hydroxycarboxyliques à partir de cellules de bactéries Gram négatives les contenant, grâce à l'expression d'un gène de lyse cloné, naturel ou chimère, caractérisé en ce qu'on augmente la concentration ionique en cations bivalents à 0,05 à 0,5 mole/l avant l'induction de l'expression du gène de lyse, on induit l'expression du gène de lyse grâce à une augmentation de la température, on récolte les cellules et on remet les cellules encore humides en suspension dans de l'eau ou dans des solutions tampons, grâce à quoi la libération des granules de polymères apparaît grâce à une lyse spontanée.

2. Procédé selon la revendication 1, caractérisé en ce qu'on utilise, comme gène de lyse, le gène lytique E cloné des bactériophages Phi X174.

3. Procédé selon la revendication 1 ou 2, caractérisé en ce qu'on augmente la concentration ionique en cations bivalents à 0,1 à 0,3 mole/l.

4. Procédé selon l'une des revendications 1 à 3, caractérisé en ce qu'on augmente la concentration ionique en cations bivalents grâce à l'addition de MgSO₄ ou CaCO₃.

5. Procédé selon l'une des revendications 1 à 4, caractérisé en ce qu'on utilise, comme bactéries Gram négatives, *Alcaligenes* ou *E. coli,* dans lesquelles des enzymes qui produisent des homo- et/ou des copolymères de l'acide 3-hydroxybutyrique et/ou de l'acide 3-hydroxyvalérianique sont clonées.

6. Procédé selon la revendication 5, caractérisé en ce qu'on utilise *E. coli* comme bactéries Gram négatives, dans lesquelles des enzymes qui produisent des homo- et/ou des copolymères de l'acide 3-hydroxybutyrique et/ou de l'acide 3-hydroxyvalérianique sont clonées.
